# EUROPEAN PATENT APPLICATION

(11) **EP 4 781 977 A1**
(43) Date of publication of application: **29.07.2026**
(21) Application number: 26150465.8
(22) Date of filing: 07.01.2026
(51) Int. Cl.: A61K 9/00, A61K 38/26, A61K 47/10, A61K 47/14, A61K 47/24, A61P 3/10

(54) **INJECTABLE FORMULATION FOR USE IN TREATING METABOLIC DISEASE AND ADMINISTRATION DEVICE**

(30) Priority: 16.01.2025 US 202563745805 P; 23.12.2025 US 202519430321
(71) Applicant: Nang Kuang Pharmaceutical Co., Ltd., Tainan City 712 (TW)
(72) Inventor: YU, Ming, 712 TAINAN CITY (TW); HSU, Ming-Hong, 712 TAINAN CITY (TW); LIN, Wei Fan, 712 TAINAN CITY (TW); CHEN, Pen Chung, 712 TAINAN CITY (TW)
(74) Representative: Buzzi, Notaro & Antonielli d'Oulx S.p.A.

(57) **Abstract**

The present invention relates to an injectable formulation for use in treating a metabolic disease and an administration device. The injectable formulation comprises a lipid matrix composition, a specific amount of a GLP-1 receptor agonist and at least one pharmaceutically acceptable ion. Accordingly, upon contacting an aqueous fluid, the injectable formulation forms a depot exhibiting an extended release of the GLP-1 receptor agonist in vivo or in vitro. The injectable formulation for use in treating the metabolic disease can be administered to a subject with a reduced dosing frequency, and it is not necessary to store the injectable formulation for the next administration. For the administration device, the injectable formulation is filled in a vial, a pre-filled syringe or a pre-filled cartridge. Therefore, the injectable formulation can be designed for a single use, thereby enhancing convenience, compliance and adherence of a subject.

## Description

### BACKGROUND

### Field of Invention

The present invention relates to an injectable formulation, a method for treating a metabolic disease and an administration device. In particular, the injectable formulation comprises a lipid matrix composition, a specific amount of a GLP-1 (Glucagon-Like Peptide-1) receptor agonist and at least one pharmaceutically acceptable ion, providing a depot formed by the injectable formulation after contacting an aqueous fluid. The depot is characterized by an extended release of the GLP-1 receptor agonist.

### Description of Related Art

A Glucagon-Like Peptide-1 (GLP-1) receptor agonist is used to treat type 2 diabetes mellitus, obesity and relative metabolic diseases. In detail, the GLP-1 receptor agonist in pancreatic β-cells increases the body weight and decreases apoptosis and drives insulin secretion in a glucose-dependent manner that decreases the risk of hypoglycemic episodes. The GLP-1 receptor agonist does not increase insulin secretion when the blood glucose is low. The GLP-1 receptor agonist also acts on pancreatic α-cells, resulting in inhibition of glucagon secretion. The GLP-1 receptor agonist also mimics the natural hormone released in response to food intake. It delays gastric emptying and helps regulation of blood glucose levels by moderating the rate at which nutrients move from the stomach to the small intestine. Besides, by inducing central satiety, the GLP-1 receptor agonist reduces food intake and results in body weight loss.

However, the GLP-1 receptor agonist is degraded by dipeptidyl peptidase-4 (DPP-4) in bloodstream and tissue of the subject.. Consequently, an acting duration of the GLP-1 receptor agonist becomes short. For this reason, a conventional injectable formulation comprising the GLP-1 receptor agonist is administered to the subject once daily or weekly, in order to maintain its concentration in blood within a therapeutic efficient range.

Besides, the conventional injectable formulation is administered in accordance with a defined dosing schedule. Regimens require more frequent administration, such as daily or weekly administration, and it tends to reduce compliance and adherence of patient over time.

In view of the above shortcoming, it is necessary to develop a new injectable formulation comprising the GLP-1 receptor agonist, a new method for treating a metabolic disease and a new administration device to solve the aforementioned drawbacks.

### SUMMARY

An aspect of the present invention is to provide an injectable formulation for a single administration. The injectable formulation comprises phosphatidyl choline, diacyl lipid, at least one biocompatible organic solvent, a specific amount of GLP-1 receptor agonist and at least one pharmaceutically acceptable ion. Upon contacting an aqueous fluid, the injectable formulation forms a depot. The depot exhibits an extended release of the GLP-1 receptor agonist in vivo or in vitro. As a result, dosing frequency of the injectable formulation is reduced, and it is not necessary to store the injectable formulation for the next administration. Accordingly, the injectable formulation can be designed for a single use, thereby enhancing convenience, compliance and adherence of a subject.

Another aspect of the present invention is to provide a method for treating a metabolic disease. In the method for treating the metabolic disease, a dosing frequency of the injectable formulation can be reduced due to formation of the depot.

A yet another aspect of the present invention is to provide an administration device. The administration device comprises a vial, a pre-filled syringe or a pre-filled cartridge filled with the injectable formulation. Due to the reduced dosing frequency of the injectable formulation, the injectable formulation can be designed for a single use, thereby enhancing convenience of patient.

According to an aspect of the present invention, an injectable formulation for a single administration is provided. The injectable formulation comprises phosphatidyl choline, diacyl lipid, at least one biocompatible organic solvent, a GLP-1 receptor agonist and at least one pharmaceutically acceptable ion. The at least one biocompatible organic solvent is selected from the group consisting of propylene glycol, N-methyl-2-pyrrolidone, ethanol and glycerol. The at least one pharmaceutically acceptable ion is selected from the group consisting of alkali metal ion and phosphate ion. A concentration of the GLP-1 receptor agonist is in a range of 0.08 wt. % to 5.0 wt. % based on 100 wt. % by weight percentage of the injectable formulation. The injectable formulation forms a depot upon contacting an aqueous fluid.

In one embodiment, the phosphatidyl choline is soybean phosphatidylcholine.

In one embodiment, the diacyl lipid is glycerol dioleate.

In one embodiment, the concentration of the GLP-1 receptor agonist is in the range of 0.1 wt. % to 4.0 wt. % based on 100 wt. % by weight percentage of the injectable formulation.

In another embodiment, an isoelectric point of the GLP-1 receptor agonist is more than 3 and less than 7.

In yet another embodiment, the GLP-1 receptor agonist has a peptide backbone and a side chain bonded thereto, the peptide backbone consists of 28 to 40 amino acids, and the side chain has a structure represented by formula I

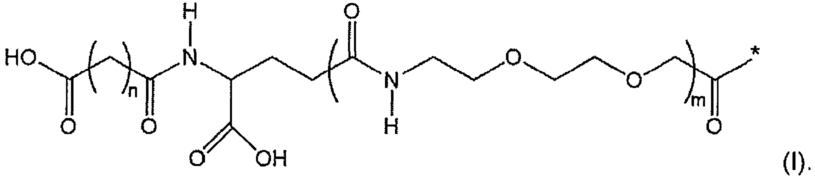

In the formula I, n is an integer from 16 to 24, m is an integer from 0 to 3, * is an attachment point connecting a carbonyl group of the side chain to an amino group of one amino acid of the 28 to 40 amino acids in the peptide backbone.

In yet another embodiment, n is an integer from 16 to 20.

In yet another embodiment, m is an integer from 0 to 2.

In yet another embodiment, the one amino acid bearing the amino group connected to the side chain is lysine.

In yet another embodiment, the GLP-1 receptor agonist is liraglutide, semaglutide, bofanglutide, tirzepatide or ecnoglutide.

In yet another embodiment, the alkali metal ion is selected from the group consisting of sodium ion, potassium ion and lithium ion.

In yet another embodiment, a mole ratio of the alkali metal ion to the GLP-1 receptor agonist is more than zero and not more than 25.

In yet another embodiment, a mole ratio of the phosphate ion to the GLP-1 receptor agonist is 0.01 to 1.2.

In yet another embodiment, the at least one pharmaceutically acceptable ion is the alkali metal ion and the phosphate ion.

In yet another embodiment, the alkali metal ion is sodium ion.

In yet another embodiment, a mole ratio of the sodium ion to the GLP-1 receptor agonist is 4 to 25.

In yet another embodiment, the injectable formulation for the single administration further compromises organic acid ion with a molecular weight less than 200 g/mole, halide ion, an alkaline amino acid or a combination thereof.

In yet another embodiment, the organic acid ion is selected from the group consisting of acetate ion, tartrate ion and citrate ion.

In yet another embodiment, the organic acid ion is the tartrate ion, the citrate ion or a combination thereof.

In yet another embodiment, a mole ratio of the acetate ion to the GLP-1 receptor agonist is not more 2.7.

In yet another embodiment, a mole ratio of the organic acid ion to the GLP-1 receptor agonist is 0.1 to 5.

In yet another embodiment, the halide ion is selected from the group consisting of chloride ion, bromide ion and iodide ion.

In yet another embodiment, a mole ratio of the halide ion to the GLP-1 receptor agonist is 0.1 to 20.

In yet another embodiment, a mole ratio of chloride ion to the GLP-1 receptor agonist is not more than 20.

In yet another embodiment, a weight ratio of the diacyl lipid to the phosphatidyl choline is from 0.5 to 1.8.

In yet another embodiment, a weight ratio of the phosphatidyl choline to the GLP-1 receptor agonist is from 10 to 85.

In yet another embodiment, a weight ratio of the diacyl lipid to the GLP-1 receptor agonist is from 3 to 110.

In yet another embodiment, a concentration of the at least one biocompatible organic solvent is in a range of 10 wt. % to 35 wt. % based on 100 wt. %by weight percentage of the injectable formulation.

In yet another embodiment, a concentration of propylene glycol is in a range of 2 wt. % to 10 wt. % based on 100 wt. % by weight percentage of N-methyl-2-pyrrolidone.

In yet another embodiment, a concentration of propylene glycol is in a range of 80 wt. % to 120 wt. % based on 100 wt. % by weight percentage of ethanol.

In yet another embodiment, the alkaline amino acid is selected from the group consisting of histidine, lysine and arginine.

In yet another embodiment, a mole ratio of the alkaline amino acid to the GLP-1 receptor agonist is 0.1 to 2.0.

In yet another embodiment, the injectable formulation further comprises water, wherein a concentration of the water is in a range of zero wt. % to 1.5 wt. % based on 100 wt. % by weight percentage of the injectable formulation.

According to another aspect of the present invention, a method for treating a metabolic disease is provided. In the method for treating the metabolic disease, an injectable formulation for a single administration is administered to a subject once every 20 to 45 days, wherein the injectable formulation comprises a lipid matrix composition, a GLP-1 receptor agonist and at least one pharmaceutically acceptable ion. The at least one pharmaceutically acceptable ion is selected from the group consisting of alkali metal ion and phosphate ion. An amount of the GLP-1 receptor agonist is 1.0 mg to 20 mg.

In one embodiment, an amount of the GLP-1 receptor agonist is 10 mg to 20 mg.

In another embodiment, the metabolic disease is selected from the group consisting of type 1 diabetes mellitus, type 2 diabetes mellitus, obesity, non-alcoholic steatohepatitis (NASH), non-alcoholic fatty liver disease (NAFLD) and cardiovascular diseases.

In yet another embodiment, the lipid matrix composition is composed of phosphatidyl choline, diacyl lipid, and at least one biocompatible organic solvent.

In yet another embodiment, the injectable formulation is administered by subcutaneous injection, intramuscular injection or intradermal injection.

In yet another embodiment, the method for treating the metabolic disease further comprises administering the GLP-1 receptor agonist to the subject in a titration dose before administration of the injectable formulation for the single administration. The titration dose is less than the amount of the GLP-1 receptor agonist in the injectable formulation for the single administration.

In yet another embodiment, the amount of the GLP-1 receptor agonist is 10 mg to 20 mg.

In yet another embodiment, an isoelectric point of the GLP-1 receptor agonist is more than 3 and less than 7.

In yet another embodiment, the GLP-1 receptor agonist has a peptide backbone and a side chain bonded thereto, the peptide backbone consists of 28 to 40 amino acids, the side chain has a structure represented by formula I

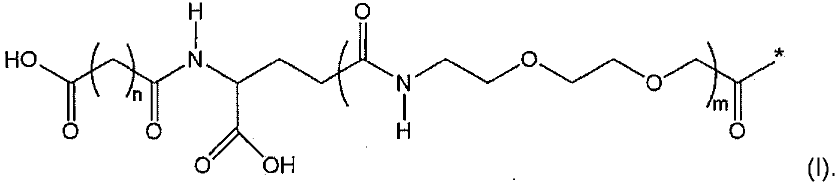

In the formula I, n is an integer from 16 to 24, m is an integer from 0 to 3, * is an attachment point connecting a carbonyl group of the side chain to an amino group of the one amino acid of the 28 to 40 amino acids in the peptide backbone.

In yet another embodiment, n is an integer from 16 to 20.

In yet another embodiment, m is an integer from 0 to 2.

In yet another embodiment, the one amino acid bearing the amino group connected to the side chain is lysine.

In yet another embodiment, the GLP-1 receptor agonist is liraglutide, semaglutide, bofanglutide, tirzepatide or ecnoglutide.

In yet another embodiment, the alkali metal ion is selected from the group consisting of sodium ion, potassium ion and lithium ion.

In yet another embodiment, a mole ratio of the alkali metal ion to the GLP-1 receptor agonist is more than zero and not more than 25.

In yet another embodiment, a mole ratio of the phosphate ion to the GLP-1 receptor agonist is 0.01 to 1.2.

In yet another embodiment, the injectable formulation for the single administration further compromises organic acid ion with a molecular weight less than 200 g/mole, halide ion, an alkaline amino acid or a combination thereof.

In yet another embodiment, the organic acid ion is selected from the group consisting of acetate ion, tartrate ion and citrate ion.

In yet another embodiment, the mole ratio of the organic acid ion to the GLP-1 receptor agonist is 0.1 to 5.

In yet another embodiment, the halide ion is selected from the group consisting of chloride ion, bromide ion and iodide ion.

In yet another embodiment, a mole ratio of the halide ion to the GLP-1 receptor agonist is 0.1 to 20.

In yet another embodiment, a weight ratio of the diacyl lipid to the phosphatidyl choline is from 0.5 to 1.8.

In yet another embodiment, a concentration of the at least one biocompatible organic solvent is in a range of 10 wt. % to 35 wt. % based on 100 wt. % by weight percentage of the injectable formulation.

In yet another embodiment, the alkaline amino acid is selected from the group consisting of histidine, lysine and arginine.

In yet another embodiment, a mole ratio of the alkaline amino acid to the GLP-1 receptor agonist is 0.1 to 2.0.

In yet another embodiment, the injectable formulation further comprises water, wherein a concentration of the water is in a range of zero wt. % to 1.5 wt. % based on 100 wt. % by weight percentage of the injectable formulation.

In yet another embodiment, the GLP-1 receptor agonist is administered to the subject in a titration dose before the injectable formulation for the single administration is administered, wherein the titration dose is less than the amount of the GLP-1 receptor agonist in the injectable formulation for the single administration.

In yet another embodiment, a weight ratio of the titration dose to the amount of the GLP-1 receptor agonist in the injectable formulation is 0.01 to 0.7.

According to yet another aspect of the present invention, use of the above-mentioned injectable formulation for manufacturing a pharmaceutical composition of treating a metabolic disease is provided.

According to yet another aspect of the present invention, a pharmaceutical composition including the above-mentioned injectable formulation for treating a metabolic disease is provided.

According to yet another aspect of the present invention, the above-mentioned injectable formulation for use in treating a metabolic disease is provided.

According to yet another aspect of the present invention, an administration device is provided. The administration device comprises a vial, a pre-filled syringe or a pre-filled cartridge filled with an injectable formulation for a single administration. The injectable formulation comprises a lipid matrix composition, a GLP-1 receptor agonist and at least one pharmaceutically acceptable ion. The at least one pharmaceutically acceptable ion is selected from the group consisting of alkali metal ion and phosphate ion. A concentration of the GLP-1 receptor agonist is in a range of 0.08 wt. % to 5.0 wt. % based on 100 wt. % by weight percentage of the injectable formulation.

In one embodiment, the concentration of the GLP-1 receptor agonist is in the range of 0.1 wt. % to 4.0 wt. % based on 100 wt. % by weight percentage of the injectable formulation.

In another embodiment, the lipid matrix composition is composed of phosphatidyl choline, diacyl lipid and at least one biocompatible organic solvent.

In yet another embodiment, the vial, the pre-filled syringe and the pre-filled cartridge are made of glass or of plastics material.

In yet another embodiment, the plastics material is cyclo olefin polymer (COP), cyclic olefin copolymers (COC), polypropylene (PP), polyethylene (PE) or a mixture thereof.

In an application of the injectable formulation for the single administration of the present invention, the injectable formulation comprises the lipid matrix composition, the specific amount of the GLP-1 receptor agonist and the at least one pharmaceutically acceptable ion, resulting in formation of depot with an extended release of the GLP-1 receptor agonist in vivo or in vitro. Accordingly, the dosing frequency of the injectable formulation can be reduced in the method for treating the metabolic disease of the present invention. Besides, it is not necessary to store the injectable formulation for the next administration. Accordingly, the injectable formulation can be designed for a single use. For the administration device, the injectable formulation is filled in the vial, the pre-filled syringe or the pre-filled cartridge, thereby improving convenience, compliance and adherence of the patient.

### BRIEF DESCRIPTION OF THE DRAWINGS

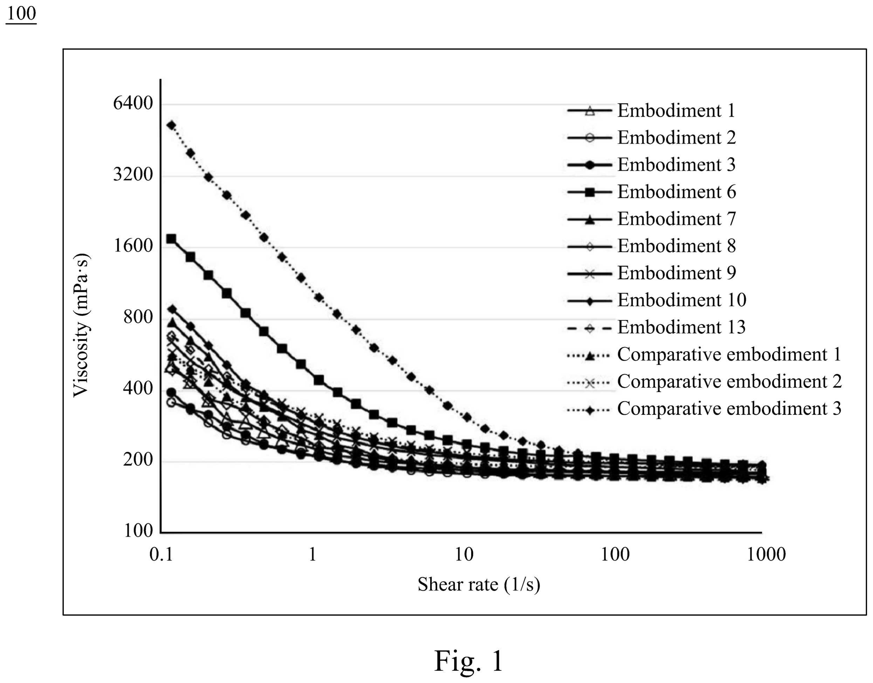

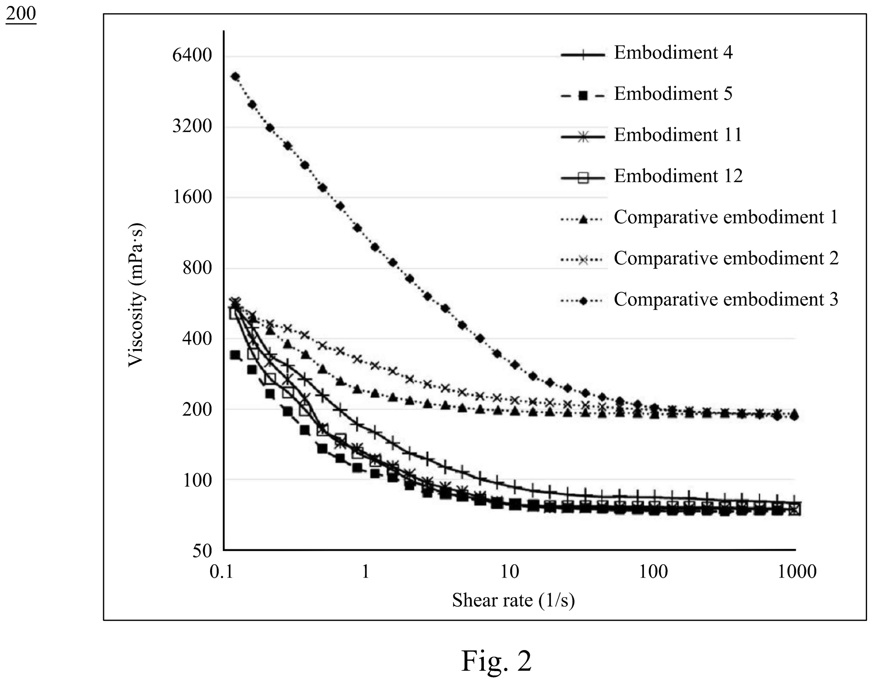

Fig. 1 to Fig. 2 are graphs illustrating viscosity of the injectable formulations in some embodiments and some comparative embodiments, obtained at 20 °C and in a shear rate range of 0.1 to 1000 s⁻¹, in which Fig. 1 includes embodiments 1-3, embodiments 6-10, embodiment 13 and comparative embodiments 1-3, and Fig. 2 includes embodiments 4-5, embodiments 11-12 and comparative embodiments 1-3.

### DETAILED DESCRIPTION

Reference will now be made in detail to the present embodiments of the invention, examples of which are illustrated in the accompanying drawings.

### Description

In an aspect of the present invention, an injectable formulation for a single administration is provided. The injectable formulation comprises a lipid matrix composition, a GLP-1 receptor agonist and at least one pharmaceutically acceptable ion. The lipid matrix composition is composed of phosphatidyl choline, diacyl lipid and at least one biocompatible organic solvent. Upon contacting an aqueous fluid, the injectable formulation forms a depot. The depot serves to release the GLP-1 receptor agonist in vivo or in vitro over an extended period.

A term "a single administration" as used herein refers to administrating the injectable formulation to a subject in need thereof once for a single use. After dosing, the injectable formulation is finished and thrown away. It is not necessary to store the injectable formulation for the next administration.

A term "depot" as used herein refers to a gel with a liquid crystalline structure. In detail, when the injectable formulation contacts the aqueous fluid, the biocompatible organic solvent in the injectable formulation disperses into the aqueous fluid and water in the aqueous fluid diffuses into the injectable formulation. Accordingly, the lipid matrix composition transforms to a lipid matrix wherein the GLP-1 receptor agonist is encapsulated, resulting in a formation of the gel with the liquid crystalline structure.

A term "aqueous fluid" as used herein refers to a fluid containing water, such as a tissue fluid of the subject or an artificial aqueous solution that mimics physiological conditions, where the GLP-1 receptor agonist is expected to be exposed. For example, water, saline and a buffer solution. Particularly, the buffer can include, but is not limited to, phosphate buffer, citrate buffer, tris buffer and any buffer solution with 5 to 8 of pH value understood by person who has the ordinary skill in the art. On the other hand, the tissue fluid of the subject can include, but is not limited to, subcutaneous fluid, intramuscular fluid or intradermal fluid of mammal animals, such as human, dogs, monkeys, rabbits, rats, mice and pigs.

A term "release in vivo" and a term "in-vivo release" as used herein refer to a release of the GLP-1 receptor agonist from the depot located within a body or a body cavity of the subject, wherein the depot is exposed to the tissue fluid of the subject, and such as subcutaneous fluid of the rat. Accordingly, the in-vivo release of the GLP-1 receptor agonist from the depot is evaluated in an animal study by recording its pharmacokinetic profile following administration of the injectable formulation.

Alternatively, a term "release in vitro" and a term "in-vitro release" as used herein refer to a release of the GLP-1 receptor agonist from the depot located within the artificial aqueous solution (hereinafter referred to as dissolution medium) that mimics physiological conditions, where the GLP-1 receptor agonist is expected to be exposed. For example, saline or a phosphate buffer without or with surfactant and/or enzyme. Accordingly, the in-vitro release of the GLP-1 receptor agonist from the depot is evaluated by measuring the residual rate of semaglutide retained within the depot after immersion in the artificial aqueous solution that mimics physiological conditions over a defined period.

A term "extended release" as used herein refers to an in-vivo or in-vitro release of the GLP-1 receptor agonist from the depot over at least than 20 days.

For the lipid matrix composition, in some embodiments, phosphatidyl choline (PC) can be derived from a natural source. For example, suitable sources of phospholipids include animal sources and plant sources, such as egg yolk, bovine heart or soybean. Any single phosphatidyl choline or a mixture of phosphatidyl choline from these or other sources can be used. Preferably, soybean phosphatidyl choline (SPC) is used in the injectable formulation in order to extend the release period of the GLP-1 receptor agonist. In other embodiment, the injectable formulation of the present invention excludes dioleoyl phosphatidyl ethanolamine (DOPE).

In preferable embodiments, phosphatidyl choline (PC) is a kind of phospholipid with a glycerol backbone, two fatty acid chains and a phosphor choline head group. In some embodiments, a chemical structure of the PC preferably comprises the two fatty acid chains with 16 to 20 carbon atoms. The fatty acid chains can be unsaturated or saturated. The fatty acid chains independently have 17 to 19 carbon atoms and 0 to 2 double bonds. In other embodiments, dodecyl phosphatidylcholine (DPC) can be used in the injectable formulation.

For instant, the concentration of the phosphatidyl choline can be 160 mg/mL to 300 mg/mL, 160 mg/mL to 380 mg/mL, 160 mg/mL to 220 mg/mL, 170 mg/mL to 250 mg/mL, 175 mg/mL to 220 mg/mL, 180 mg/mL to 200 mg/mL, 180 mg/mL to 250 mg/mL, 180 mg/mL to 230 mg/mL, 180 mg/mL to 340 mg/mL, 190 mg/mL to 220 mg/mL, 200 mg/mL to 260 mg/mL, 200 mg/mL to 360 mg/mL, 200 mg/mL to 340 mg/mL, 195 mg/mL to 280 mg/mL, 220 mg/mL to 260 mg/mL, 220 mg/mL to 380 mg/mL, 220 mg/mL to 330 mg/mL, 300 mg/mL to 380 mg/mL or any value within the aforementioned range. When the concentration of the phosphatidyl choline is within the aforementioned range, the gel is easily formed, thereby extending the release period of the GLP-1 receptor agonist.

The diacyl lipid is a kind of lipid composed of one glycerol molecule bonded to two fatty acid molecules through ester linkages. In some embodiments, the two fatty acid molecules can be saturated or unsaturated and independently include alkyl and/or alkenyl groups with 16 to 20 carbon numbers. Preferably, glycerol dioleate (GDO) is used in the injectable formulation to extend the release period of the GLP-1 receptor agonist.

In some embodiments, a weight ratio of the diacyl lipid to the phosphatidyl choline is from 0.5 to 1.8, such as from 0.6 to 1.8, from 0.9 to 1.6, from 0.5 to 0.8 or any value within the aforementioned range. When the weight ratio of the diacyl lipid to the phosphatidyl choline is from 0.5 to 1.8, the gel is easily formed, thereby extending the release period of the GLP-1 receptor agonist.

The at least one biocompatible organic solvent is selected from the group consisting of propylene glycol (PG), N-methyl-2-pyrrolidone (NMP), ethanol and glycerol. In some embodiments, a concentration of the at least one biocompatible organic solvent is in a range of 10 wt. % to 35 wt. % based on 100 wt. by weight percentage of the injectable formulation, such as 10 wt. %, 15 wt. %, 20 wt. %, 25 wt. %, 30 wt. %, 35 wt. % or any value within the aforementioned range.

In another embodiments, the concentration of propylene glycol is in a range of 2 wt. % to 10 wt. % based on 100 wt. % by weight percentage of N-methyl-2-pyrrolidone, such as 2 wt. %, 4 wt. %, 6 wt. %, 8 wt. %, 10 wt. % or any value within the aforementioned range. In yet another embodiment, the concentration of propylene glycol is in a range of 80 wt. % to 120 wt. % based on 100 wt. % by weight percentage of ethanol, such as 80 wt. %, 90 wt. %, 100 wt. %, 110 wt. %, 120 wt. % or any value within the aforementioned range. When the at least one biocompatible organic solvent meets the pervious conditions, the gel is easily formed, thereby extending the release period of the GLP-1 receptor agonist.

In yet another embodiment, a concentration of glycerol is in a range of 0.1 wt. % to 8 wt. % based on 100 wt. % by weight percentage of the injectable formulation, and preferably 0.5 wt. % to 5 wt. %. When the concentration of glycerol meets the pervious conditions, the gel is easily formed, thereby extending the release period of the GLP-1 receptor agonist.

Moreover, the injectable formulation of the present invention has 1.0 mg to 20 mg of the GLP-1 receptor agonist. If the amount of the GLP-1 receptor agonist is less than 1.0 mg, the release period of the GLP-1 receptor agonist is reduced. On contrast, if the amount of the GLP-1 receptor agonist is more than 20 mg, the initial burst release of GLP-1 receptor agonist from the gel is large, causing toxicity to the subject. In preferable embodiments, the amount of the GLP-1 receptor agonist is 10 mg to 20 mg, and more preferably 10 mg to 15 mg.

In some embodiments, an isoelectric point of the GLP-1 receptor agonist is more than 3 and less than 7, and preferably 4 to 6. When the isoelectric point of the GLP-1 receptor agonist is within the aforementioned range, the release period of the GLP-1 receptor agonist can be extended.

In general, the GLP-1 receptor agonist has a peptide backbone and a side chain bonded thereto. In some embodiments, the peptide backbone consists of 28 to 40 amino acids. The side chain has a structure represented by the following formula I:

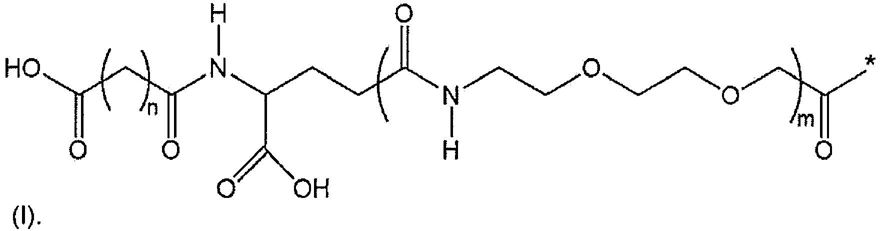

In the formula I, n is an integer from 16 to 24, m is an integer from 0 to 3, and * is an attachment point connecting a carbonyl group of the side chain to an amino group of the one amino acid of the 28 to 40 amino acids in the peptide backbone.

Preferably, n is an integer from 16 to 20 and / or m is an integer from 0 to 2. When n and / or m is one of the aforementioned integers, the release period of the GLP-1 receptor agonist can be extended.

In some examples, the one amino acid bearing the amino group connected to the side chain is lysine. When the one amino acid bearing the amino group connected to the side chain meets the pervious conditions, the release period of the GLP-1 receptor agonist can be extended.

In preferable embodiments, the GLP-1 receptor agonist is liraglutide, semaglutide, bofanglutide, tirzepatide or ecnoglutide. When the GLP-1 receptor agonist meets the pervious conditions, the release period of the GLP-1 receptor agonist can be extended. Preferably, the GLP-1 receptor agonist has a critical micelle concentration of 0.06 wt. % in an aqueous solution.

In specific examples, a concentration of the semaglutide can be 10 mg/mL to 35 mg/mL, such as 15 mg/mL to 25 mg/mL, 15 mg/mL to 20 mg/mL, 20 mg/mL to 25 mg/mL or any value within aforementioned range. In alternative examples, the concentration of the semaglutide can be 10, 15, 20, 25, 35 mg/mL or any value within aforementioned range. Alternatively, the concentration of the semaglutide can be in a range of 1.5 wt. % to 4.5 wt. % or any value within aforementioned range.

In other specific examples, a concentration of the semaglutide can be 0.5 mg/mL to 25 mg/mL, such as 2 mg/mL to 20 mg/mL, 3 mg/mL to 15 mg/mL, 4 mg/mL to 10 mg/mL or any value within aforementioned range. In alternative examples, the concentration of the semaglutide can be 0.5, 2, 3, 4, 10, 15, 20, 25 mg/mL or any value within aforementioned range. Alternatively, the concentration of the semaglutide can be in a range of 0.08 wt. % to 5.0 wt. % based on 100 wt. % by weight percentage of the injectable formulation, such as 0.1 wt. % to 4.0 wt. %, 0.2 wt. % to 3.0 wt. %, 0.3 wt. % to 2.5 wt. %, 0.4 wt. % to 2.0 wt. % or any value within aforementioned range. For example, the concentration of the semaglutide is 0.08 wt. %, 0.1 wt. %, 0.2 wt. %, 0.3 wt. %, 0.4 wt. %, 0.5 wt. %, 0.6 wt. %, 0.7 wt. %, 0.8 wt. %, 0.9 wt. %, 1.0 wt. %, 2.0 wt. %, 3.0 wt. %, 4.0 wt. %, 5.0 wt. % or any value within aforementioned range.

The GLP-1 receptor agonist can enhance glucose-stimulated insulin secretion and biosynthesis, suppress glucagon secretion, slow gastric emptying, and reduce food intake and appetite. These actions collectively contribute to blood sugar regulation and can lead to body weight loss. Consequently, the GLP-1 receptor agonist can reduce cardiovascular risk, reduce body weight and improve lipid profiles in patients with type 2 diabetes.

In some embodiments, the GLP-1 receptor agonist of the present invention can also function as GIP (glucose-dependent insulinotropic polypeptide) receptor agonist, specifically referred to as GLP-1/GIP receptor dual agonists. In some examples, the GLP-1/GIP receptor dual agonists can include tirzepatide. In relative examples, an amount of tirzepatide in the injectable formulation is more than 15 mg and less than 60 mg, 20 mg to 55 mg, 25 mg to 50 mg, or 30 mg to 90 mg.

The at least one pharmaceutically acceptable ion is selected from the group consisting of alkali metal ion and phosphate ion. In some embodiments, the alkali metal ion is selected from the group consisting of sodium ion, potassium ion and lithium ion. Preferably, sodium ion is used in the injectable formulation. When sodium ion, potassium ion or lithium ion is present in the injectable formulation, the release period of the GLP-1 receptor agonist from the gel can be extended.

The alkali metal ion can be introduced into the injectable formulation by using salt of corresponding alkali metal or hydroxide of the alkali metal. The salt of corresponding alkali metal can be, not limited to, alkali metal halide or alkali metal salt of weak organic acid or weak inorganic acid. Preferably, the weak organic acid and the weak inorganic acid have good buffer capacity to resistance to pH changes.

In alternative embodiments, hydrogen atoms in carboxylic acid groups of the GLP-1 receptor agonist are partially or completely replaced by the alkali metal ion via ion exchange techniques, such as by using ion exchange resins. In relative examples, the alkali metal ion comes from a buffer solution of the salt of the corresponding alkali metal dissolved in a polar solvent, and such as water.

Moreover, in preferable examples, a mole ratio of the alkali metal ion to the GLP-1 receptor agonist is more than zero and not more than 25, preferably 1 to 22, and more preferably 1 to 11. For example, the mole ratio of the alkali metal ion to the GLP-1 receptor agonist is 1, 2, 3, 4, 5, 6, 7, 8, 9, 10, 11, 12, 13, 14, 15, 16, 17, 18, 19, 20, 21, 22, 23, 24, 25 or any value within the aforementioned range. When the mole ratio of the alkali metal ion to the GLP-1 receptor agonist meets the pervious conditions, the release period of the GLP-1 receptor agonist can be extended.

Alternatively, in other examples, a mole ratio of the phosphate ion to the GLP-1 receptor agonist is 0.01 to 1.2, preferably 0.01 to 1.0, and more preferably 0.04 to 0.9. For example, the mole ratio of the phosphate ion to the GLP-1 receptor agonist is 0.01, 0.05, 0.1, 0.2, 0.3, 0.4, 0.5, 0.6, 0.7, 0.8, 0.9, 1.0 or any value within the aforementioned range. When mole ratio of the phosphate ion to the GLP-1 receptor agonist meets the pervious conditions, the release period of the GLP-1 receptor agonist can be extended.

In preferable examples, the pharmaceutically acceptable ion is the alkali metal ion (e.g., sodium ion) and the phosphate ion, and a mole ratio of the alkali metal ion and the phosphate ion is 1 to 300, such as 1, 5, 10, 20, 30, 40, 50, 60, 70, 80, 90, 100, 110, 130,150, 170, 190, 200, 210, 230, 250, 270, 290 or any value within the aforementioned range. Within the abovementioned mole ratio, suitable viscosity of the injectable formulation can be achieved, and an intact appearance of the gel formed by the injectable formulation can also be achieved.

The organic acid ion and phosphate ion can be introduced into the injectable formulation by using corresponding acids or salts thereof, such as acetic acid, citric acid, phosphoric acid, sodium acetate, sodium citrate, disodium hydrogen phosphate and sodium dihydrogen phosphate. For example, these ions can be directly applied to the injectable formulation as a buffer solution of the salts thereof or indirectly as the corresponding acids.

As understood by person who has the ordinary skill in the art, phosphoric acid is partially or not completely ionized in an aqueous solution, depending a pH value of the aqueous solution, wherein the phosphoric acid can generate phosphate ion. Consequently, the phosphate ion of the present invention includes orthophosphate ion (PO₄³⁻), hydrogen phosphate ion (HPO₄²⁻) and dihydrogen phosphate ion (H₂PO₄⁻). Accordingly, the amount of the phosphate ion of the present invention is a total of these above-mentioned ions.

In yet another embodiment, the injectable formulation for the single administration can further compromises organic acid ion with a molecular weight less than 200 g/mole, halide ion, an alkaline amino acid or a combination thereof.

In some embodiments, the organic acid ion with a molecular weight less than 200 g/mole is selected from the group consisting of acetate ion, tartrate ion and citrate ion. When acetate ion, tartrate ion or citrate ion is used in the injectable formulation, the release period of the GLP-1 receptor agonist can be extended.

Specifically, in some examples, a mole ratio of the organic acid ion to the GLP-1 receptor agonist is 0.1 to 5, preferably 0.1 to 2.5, and more preferably 0.1 to 0.95. For example, the mole ratio of the organic acid ion to the GLP-1 receptor agonist is 0.1, 0.5, 1.0, 1.5, 2.0, 2.5 or any value within the aforementioned range. When the mole ratio of the organic acid ion to the GLP-1 receptor agonist meets the pervious conditions, the release period of the GLP-1 receptor agonist can be extended.

In some preferred examples, the organic acid ion is the tartrate ion, the citrate ion or a combination thereof, and the acetate ion is excluded. In some examples, a mole ratio of the acetate ion to the GLP-1 receptor agonist is not more than 2.7, such as 0.01, 0.1, 0.25, 0.5, 0.75, 1.0, 1.25, 1.5, 1.75, 2.0, 2.5, 2.6 or any value within the aforementioned range.

In another embodiments, the halide ion is selected from the group consisting of chloride ion, bromide ion and iodide ion. Preferably, the halide ion is chloride ion or bromide ion. When chloride ion, bromide ion or iodide ion is used in the injectable formulation, the release period of the GLP-1 receptor agonist can be extended.

In relative examples, a mole ratio of the halide ion to the GLP-1 receptor agonist is 0.1 to 20, preferably 0.1 to 17, and more preferably 0.1 to 7. For example, the mole ratio of the halide ion to the GLP-1 receptor agonist is 0.1, 0.5, 1.0, 1.5, 2.0, 2.5, 3.0, 3.5, 4.0, 4.5, 5.0, 5.5, 6.0, 6.5, 7.0 or any value within the aforementioned range. When the mole ratio of the halide ion to the GLP-1 receptor agonist meets the pervious conditions, the release period of the GLP-1 receptor agonist can be extended.

In some examples, the chloride ion is excluded from the injectable formulation so as to reduce viscosity. In some examples, a mole ratio of chloride ion to the GLP-1 receptor agonist is not more than 20, such as 0.01, 0.1, 0.25, 0.5, 0.75, 1.0, 4.0, 6.0, 7.0, 8.0, 10, 12, 14, 16, 17, 18, 20 or any value within the aforementioned range. Within the abovementioned mole ratio, suitable viscosity and intact appearance of the injectable formulation can be achieved.

The halide ion can be introduced into the injectable formulation by using salt of corresponding halide or an aqueous solution of hydrogen halide. In relative examples, the halide ion comes from the aqueous solution of the salt of the corresponding halide, such as sodium chloride, potassium chloride and sodium bromide.

As stated above, in specific examples, the alkali metal ion can be introduced into the injectable formulation in combination with organic acid ion, phosphate ion or halide ion. For example, sodium acetate, sodium citrate, sodium dihydrogen phosphate, disodium hydrogen phosphate or a combination thereof can be dissolved in a polar solvent, such as water or ethanol, and then added into the injectable formulation. In these examples, the mole ratio of the alkali metal ion to these aforementioned anions is stoichiometrically based on their corresponding salts.

However, in other examples, the mole ratio of the alkali metal ion to these aforementioned anions is not stoichiometrically based on their corresponding salts. In these relative examples, the alkali metal ion and these aforementioned anions are individually introduced into the injectable formulation, or partial of them are individually introduced into the injectable formulation. When the mole ratio of the alkali metal ion to these aforementioned anions is not stoichiometrically based on their corresponding salts, the release period of the GLP-1 receptor agonist can be extended.

In yet another embodiment, the injectable formulation for the single administration of the present invention further compromises an alkaline amino acid. Specifically, the alkaline amino acid can be selected from the group consisting of histidine, lysine and arginine. When at least one of the aforementioned alkaline amino acids is present in the injectable formulation, the release period of the GLP-1 receptor agonist from the gel can be extended.

In some examples, a mole ratio of the alkaline amino acid to the GLP-1 receptor agonist is 0.1 to 2.0, preferably 0.1 to 1.5, and more preferably 0.5 to 0.8. For example, the mole ratio of the alkaline amino acid to the GLP-1 receptor agonist is 0.1, 0.5, 1.0, 1.5, 2.0 or any value within the aforementioned range. When the mole ratio of the alkaline amino acid to the GLP-1 receptor agonist is within the aforementioned range, the release period of the GLP-1 receptor agonist from the gel is extended.

For an active agent, in preferable examples, the injectable formulation of the present invention excludes somatostatin receptor agonist, octreotide, setmelanotide, leuprolide, pasireotide, granisetron, gonadotrophin-releasing hormone (GnRH) agonist, goserelin, buprenorphine, opioid, prostacyclin, 5HT3 antagonist or any combination thereof. When the active agent of the injectable formulation excludes the aforementioned active agents, the injectable formulation can provide good therapeutical efficacy.

For excipients, in preferable examples, the injectable formulation of the present invention excludes polyethylene glycosylated fatty acids, polyethylene glycosylated phospholipids, polyethylene glycosylated glyceryl fatty acid esters, ester of a sugar or any combination thereof. When the injectable formulation of the present invention excludes the aforementioned excipients, the release period of the GLP-1 receptor agonist from the gel can be extended, or thermal stability or syringeablility of the injectable formulation can be enhanced. In other examples, the injectable formulation of the present invention excludes polysorbate 80, polysorbate 20, sugars, sugar derivative sorbitan monooleate, sorbitan dioleate, sorbitan trioleate or sorbitan tetraoleate.

In some embodiments, the injectable formulation of the present invention can further comprise water, wherein a concentration of the water is in a range of zero wt. % to 1.5 wt. %, such as 0.1 to 1.5 wt. %, 0.5 to 1.5 wt. %, 0.5 to 1.0 wt. %, or 1.0 to 1.5 wt. %, based on 100 wt. % by weight percentage of the injectable formulation. When the concentration of the water in the formulation is within the pervious range, the small amount of water can extend the release period of the GLP-1 receptor agonist. Specifically, the concentration of the water in the injectable formulation of the present invention can be determined by water content of the injectable formulation by using a Karl-Fischer titrator.

In an in-vitro release test, the injectable formulation is loaded into a dissolution medium, and then stood for twenty to thirty minutes to form a gel. The gel placed in a spinning dissolution basket is immersed in the dissolution medium for a predetermined time. Next, a residual amount of the GLP-1 receptor agonist within the gel is measured by liquid chromatography and its residual rate is calculated based on an original weight of GLP-1 receptor agonist of the injectable formulation as 100 wt. %. Assuming a sum of the residual rate and a release rate of the GLP-1 receptor agonist is equal to 100 wt. %, its release rate can also be calculated.

When the residual rate of the GLP-1 receptor agonist within the gel is more than zero percentage, it is inferred that the release period of the GLP-1 receptor agonist from the gel exceeds the predetermined timepoint associated with the measurement of the residual amount of the GLP-1 receptor agonist. In some embodiments, the residual rate of the GLP-1 receptor agonist within the gel in the dissolution medium over 20 days is more than 24 %, preferably more than 50 % and more preferably more than 60 %. In other embodiments, the residual rate of the GLP-1 receptor agonist within the gel in the dissolution medium over 31 days is more than 23.5 %, preferably more than 40 % and more preferably more than 55 %.

On the other hand, in an in-vivo release test, an animal is subcutaneously administrated with the injectable formulation, and then a pharmacokinetic profile of the GLP-1 receptor agonist in its blood is recorded, to evaluate a release period of GLP-1 receptor agonist from the gel in tissue fluid of the animal. In some embodiments, the GLP-1 receptor agonist releases in vivo from the gel over 20 days, more than 20 days, preferably more than 30 days, and more preferably more than 40 days. In some embodiments, the GLP-1 receptor agonist releases in vivo from the gel over 25 to 30 days. In other embodiments, the GLP-1 receptor agonist releases in vivo from the gel over 21 to 42 days, 21 to 35 days, 28 to 42 days, 28 to 35 days or 35 to 42 days.

The extended release of the GLP-1 receptor agonist of the present invention can extend an action duration in vivo, thereby reducing the dosing frequency of the injectable formulation. Consequently, the injectable formulation can be designed for single use. Since the injectable formulation in unit dose can be finished once, it is not necessary to store the injectable formulation until the next administration. Accordingly, the injectable formulation of the present invention can provide more convenience for the patients, enhancing compliance and adherence thereof.

Besides reduction of dosing frequency, the injectable formulation of the present invention also has excellent thermal stability. Therefore, it can be stored and transported at room temperature instead of 2 °C to 8 °C for the conventional injectable formulation. The thermal stability of the injectable formulation is estimated by an appearance of the injectable formulation and its assay of the GLP-1 receptor agonist.

In some embodiments, no precipitation or no gel is formed in the injectable formulation after undergoing six consecutive cycles of freezing at -20 °C and thawing at 25 °C. Preferably, a decrease of the assay of the GLP-1 receptor agonist in the injectable formulation is less than 17 %, after undergoing six consecutive cycles of freezing at -20 °C and thawing at 25 °C.

Moreover, the injectable formulation of the present invention has rheological property, which can enhance syringeablility. Detailly, the injectable formulation is administered easily through a thin needle, providing comfort for the patient during administration, rather than stinging and aching caused by a thick needle for the conventional injectable formulation. In some embodiments, at 20 °C, the injectable formulation has a larger viscosity in the shear rate range of 0.1 to 0.5 s⁻¹ than that in the shear rate range of 100 to 1000 s⁻¹. In specific examples, the viscosity of the injectable formulation is less than 1000 mPa·s at the shear rate of 1 s⁻¹, thereby enhancing its syringeablility. For example, the viscosity of the injectable formulation is 50 mPa·s, 100 mPa·s, 200 mPa·s, 300 mPa·s, 400 mPa·s, 500 mPa·s, 600 mPa·s, 700 mPa·s, 800 mPa·s, 900 mPa·s, 950 mPa·s, or any value within the aforementioned range at the shear rate of 1 s⁻¹.

In another aspect of the present invention, an administration device is provided. The administration device comprises a vial, a pre-filled syringe or a pre-filled cartridge. The vial, the pre-filled syringe and the pre-filled cartridge are filled with an injectable formulation for a single administration. The injectable formulation comprises a lipid matrix composition, a GLP-1 receptor agonist and at least one pharmaceutically acceptable ion. The at least one pharmaceutically acceptable ion is selected from the group consisting of alkali metal ion and phosphate ion. In some embodiments, a concentration of the GLP-1 receptor agonist is in a range of 0.08 wt. % to 5.0 wt. % based on 100 wt. % by weight percentage of the injectable formulation, providing 1.0 mg to 20 mg GLP-1 receptor agonist for single use. In other embodiments, a concentration of the GLP-1 receptor agonist is in a range of 0.08 wt. % to 5.0 wt. % based on 100 wt. % by weight percentage of the injectable formulation, providing 15 mg to 90 mg GLP-1 receptor agonist for single use.

In some embodiments, the administration device can be one as well-known by person who has the ordinary skill in the art, wherein the vial, the pre-filled syringe and the pre-filled cartridge are also made of a material used by person who has the ordinary skill in the art. In another embodiments, the vial, the pre-filled syringe and the pre-filled cartridge are made of glass or of plastics material. The glass material can enhance biocompatibility of the vial, the pre-filled syringe and the pre-filled cartridge.

On the other hand, the plastics material can provide the vial, the pre-filled syringe and the pre-filled cartridge with precise dimensions due to an injection molding process in their production. Thus, a dead volume in the vial, the pre-filled syringe and the pre-filled cartridge is minimized.

Preferably, the plastics material can include, but not limited to, cyclo olefin polymer (COP), cyclic olefin copolymers (COC), polypropylene (PP), polyethylene (PE) or a mixture thereof. The cyclo olefin polymer and the cyclic olefin copolymers have good solvent-resistance, especially for biocompatible organic solvents, such as ethanol. When these plastics materials are used to produce the vial, the pre-filled syringe and the pre-filled cartridge, the vial, the pre-filled syringe and the pre-filled cartridge have precise dimensions and good biocompatibility.

In another aspect of the present invention, a method for treating a metabolic disease is provided. In the method for treating the metabolic disease, an injectable formulation for a single administration is administered to a subject once every 20 to 45 days, such as 20 days, 25 days, 30 days, 35 days, 40 days, 45 days or any value within the aforementioned range. In some embodiments, the injectable formulation is administered to the subject once every 20 to 30 days, every 30 to 40 days, every 25 to 35 days or every 35 to 45 days. In other embodiments, the injectable formulation is administered to the subject once every 21 to 42 days, 21 to 35 days, 28 to 42 days, 28 to 35 days or 35 to 42 days.

A term "subject" as used herein refers to mammal animals, such as human, dogs, monkeys, rabbits, rats, mice and pigs. In some embodiments, the subject does not suffer from medullary thyroid carcinoma, multiple endocrine neoplasia type 2 syndrome or pancreatitis.

In the method for treating the metabolic disease, the dosing frequency or the administration interval of the injectable formulation dependents on action duration in vivo of the GLP-1 receptor agonist, which can be estimated by the release of the GLP-1 receptor agonist from the depot, such as the in-vitro releases and in-vivo release of the GLP-1 receptor agonist as mentioned above.

In some embodiments, the injectable formulation can be administered to the subject with escalated dose of the GLP-1 receptor agonist sequentially. For example, the dose of the GLP-1 receptor agonist is escalated by 1 to 20 mg with each successive dose. In these examples, the subject is administered by one injectable formulation with small volume initially and then by the other injectable formulation with large volume. With vary of volume of the injectable formulation, the injectable formulation can be administered to the subject with escalated dose of the GLP-1 receptor agonist sequentially.

Generally, routes of administration of the injectable formulation can be those well-known by person who has the ordinary skill in the art. For example, the injectable formulation is administered by subcutaneous injection, intramuscular injection or intradermal injection.

In the method for treating the metabolic disease, preferably, the GLP-1 receptor agonist is administered to the subject in a titration dose before the injectable formulation is administered. The titration dose is less than the amount of the GLP-1 receptor agonist in the injectable formulation. The titration dose of the GLP-1 receptor agonist can be taken via an oral administration or other administration as well-known by person who has the ordinary skill in the art. On the other hand, the amount of the GLP-1 receptor agonist in the injectable formulation is called a maintenance dose.

In some embodiments, the titration dose is less than the maintenance dose by 1.0 mg, 1.25 mg, 1.5 mg, 1.75 mg, 2.0 mg, 2.25 mg, 2.5 mg, 2.75 mg, 3.0 mg, 3.25 mg, 3.5 mg, 3.75 mg, 4.0 mg, 4.25 mg, 4.5 mg or 4.75 mg. In other embodiments, a weight ratio of the titration dose to the maintenance dose is 0.01 to 0.7, 0.02 to 0.5, 0.3 to 0.7 or 0.25 to 0.5. When the method further comprises administration of the GLP-1 receptor agonist in the titration dose, therapeutical efficacy can be enhanced.

In relative examples, the titration dose of the GLP-1 receptor agonist can be administered once weekly for one to four weeks followed by the maintenance dose of the GLP-1 receptor agonist once monthly for subsequent administrations. In specific examples, the titration dose of the GLP-1 receptor agonist is administered once every 7 to 21 days, every 10 to 18 days, every 21 to 35 days or 24 to 31 days. In detail, the titration dose of the GLP-1 receptor agonist can be administered until a concentration of 3 to 6 ng/mL of GLP-1 receptor agonist in blood of the subject is reached.

Moreover, the titration dose of the GLP-1 receptor agonist can be administered 1 to 3 times, and its administration interval is one week to three weeks. In relative examples, the titration dose of the GLP-1 receptor agonist can be escalated sequentially. Besides, the maintenance dose of the GLP-1 receptor agonist also can be escalated sequentially. For example, the maintenance dose of the GLP-1 receptor agonist is escalated by 1 to 10 mg with each successive dose, and preferably by 5 to 10 mg.

For pharmacokinetic profile, in some embodiments, a maximum concentration (Cₘₐₓ) of the GLP-1 receptor agonist in blood of the subject is not more than 15 times of a minimum concentration (Cₘᵢₙ) of the GLP-1 receptor agonist in the blood of the subject, preferably not more than 12 times and more preferably not more than 9 times. For example, Cₘₐₓ of the GLP-1 receptor agonist in blood of the subject is not more than 2 times, not more than 3 times, not more than 4 times, not more than 5 times, not more than 6 times, not more than 7 times, not more than 8 times, not more than 9 times, not more than 10 times, not more than 11 times, or not more than 12 times of Cₘᵢₙ of the GLP-1 receptor agonist in the blood of the subject.

In preferable embodiments, a maximum concentration (Cₘₐₓ) of the GLP-1 receptor agonist in blood of the subject is not more than 9 times of a minimum concentration (Cₘᵢₙ) of the GLP-1 receptor agonist in the blood of the subject, preferably not more than 5 times and more preferably not more than 3 times. In specific examples, the Cₘₐₓ of the semaglutide in blood of the subject is not more than 9 times of the Cₘᵢₙ of the semaglutide in the blood of the subject, preferably not more than 5 times and more preferably not more than 3 times. When the Cₘₐₓ and the Cₘᵢₙ meet the pervious conditions, compliance and adherence of the subject can be enhanced.

In specific embodiments, the metabolic disease can be selected from the group consisting of type 1 diabetes mellitus, type 2 diabetes mellitus, obesity, non-alcoholic steatohepatitis (NASH), non-alcoholic fatty liver disease (NAFLD), and cardiovascular diseases. Preferably, according to indication of metabolic disease, the amount of the GLP-1 receptor agonist in the injectable formulation can be optimized.

Moreover, the above stated metabolic disease can be treated or relieved by reduction of glucose level in blood, suppression of secretion of glucagon from pancreatic alpha-cells, reduction of HbA1c level in blood and / or delay of gastric empty. In some embodiments, HbA1c level of the subject is reduced by at least 1.0 %, such as 1.0 to 3.0 %, following treatment for at least 3 months. In relative embodiments, body weight of the subject without type 2 diabetes mellitus is decreased by at least 5 %, such as 5 to 23 %, following treatment for at least 3 months. In relative embodiments, food intake of the subject is decreased by at least 20 %, such as 20 to 40 %, following treatment for at least 3 months. In relative embodiments, some metabolic parameters of the subject can be improved, such as cholesterol level and blood sugar level.

Embodiment 1 to Embodiment 15 and Comparative Embodiment 1 to Comparative Embodiment 3

### Preparation of injectable formulations

According to table 1 to table 4, in each embodiment, at room temperature (about 25°C), soybean phosphatidylcholine and glycerol dioleate were dissolved in desired solvents to create a non-aqueous solution. Then, at room temperature under nitrogen environment, semaglutide and other ingredients were added into and dissolved in the non-aqueous solution to obtain the resulted injectable formulation. The resulted injectable formulation was filled into a syringe made of glass or cyclo olefin polymer (COP). In table 1 to table 4, a term "NA" indicated that the mentioned ingredient was not used in the injectable formulation. N-methyl-2-pyrrolidone or ethanol was used as a solvent, and their added weights were adjusted so that a weight of the corresponding resulted injectable formulation reached to 100 wt. %. In all of the embodiments and all of the comparative embodiments, a concentration of the at least one biocompatible organic solvent was 10 wt. % to 35 wt. % based on 100 wt. % by weight percentage of the corresponding resulted injectable formulation. Besides, in all of the embodiments and all of the comparative embodiments, a volume of the resulted injectable formulation was 0.4 mL to 2 mL. Unless otherwise stated, the concentration of each ingredient in table 1 to table 4 was expressed based on the weight of the corresponding resulted injectable formulation being 100 wt. %.

**Table 1. injectable formulations**

| | Embodiment | | | | |
|---|---|---|---|---|---|
| | 1 | 2 | 3 | 4 | 5 |
| Semaqlutide (mg) | 2.4 | 2.4 | 2.4 | 7.2 | 13.5 |
| Semaglutide (wt. %) | 0.44 | 0.44 | 0.44 | 1.13 | 2.11 |
| Soybean phosphatidylcholine (wt. %) | 31.4 | 31.4 | 31.4 | 26.7 | 26.6 |
| Dioleoyl phosphatidyl ethanolamine (wt. %) | NA | NA | NA | NA | NA |
| Glycerol dioleate (wt. %) | 47.3 | 47.3 | 47.3 | 40.2 | 40.1 |
| Propylene glycol (wt. %) | 1.4 | 1.4 | 1.4 | 2.5 | 1.4 |
| Glycerol (wt. %) | NA | NA | NA | NA | NA |
| Other solvent | N-methyl-2-pyrrolidone | -N-methyl-2-pyrrolidone | N-methyl-2-pyrrolidone | -N-methyl-2-pyrrolidone | -N-methyl-2-pyrrolidone |
| Concentration of PG based on 100 wt. % by weight percentage of N-methyl-2-pyrrolid | 7.6 | 7.6 | 7.6 | 8.5 | 4.7 |
| Concentration of PG based on 100 wt. % by weight percentage of Ethanol | NA | NA | NA | NA | NA |
| Mole ratio of alkali metal ion to GLP-1 receptor agonist | Sodium ion | Sodium ion | Sodium ion | Sodium ion | Sodium ion |
| | 4.55 | 4.40 | 4.55 | 4.40 | 4.40 |
| Mole ratio of phosphate ion to GLP-1 receptor agonist | 0.11 | 0.04 | 0.11 | 0.04 | 0.04 |
| Mole ratio of organic acid ion to GLP-1 receptor agonist | NA | NA | NA | NA | NA |
| Mole ratio of halide ion to GLP-1 receptor agonist | NA | NA | NA | NA | NA |
| Mole ratio of amino acid to GLP-1 receptor agonist | NA | Histidine | Histidine | NA | NA |
| | | 0.88 | 0.88 | | |
| Syringe | Glass | Glass | Glass | Glass | Glass |

**Table 2. injectable formulations**

| | Embodiment | | | | |
|---|---|---|---|---|---|
| | 6 | 7 | 8 | 9 | 10 |
| Semaglutide (mg) | 2.4 | 2.4 | 2.4 | 2.4 | 5 |
| Semaglutide (wt. %) | 0.44 | 0.44 | 0.44 | 0.44 | 0.92 |
| Soybean phosphatidylcholine (wt. %) | 31.4 | 31.4 | 31.4 | 31.4 | 31.3 |
| Dioleoyl phosphatidyl ethanolamine (wt. %) | NA | NA | NA | NA | NA |
| Glycerol dioleate (wt. %) | 47.3 | 47.3 | 47.3 | 47.3 | 47.1 |
| Propylene glycol (wt. %) | 1.4 | 1.4 | 1.4 | 1.4 | 1.4 |
| Glycerol (wt. %) | NA | NA | NA | NA | NA |
| Other solvent | N-methyl-2-pyrrolidone | -N-methyl-2-pyrrolidone | N-methyl-2-pyrrolidone | -N-methyl-2-pyrrolidone | -N-methyl-2-pyrrolidone |
| Concentration of PG based on 100 wt. % by weight percentage of N-methyl-2-pyrrolid one | 7.6 | 7.6 | 7.6 | 7.6 | 7.6 |
| Concentration of PG based on 100 wt. % by weight percentage of Ethanol | NA | NA | NA | NA | NA |
| Mole ratio of alkali metal ion to GLP-1 receptor agonist | Sodium ion | Sodium ion | Sodium ion | Sodium ion | Sodium ion |
| | 22.01 | 7.05 | 6.17 | 11.89 | 5.25 |
| Mole ratio of phosphate ion to GLP-1 receptor agonist | 0.04 | 0.04 | 0.92 | 0.04 | 0.46 |
| Mole ratio of organic acid ion to GLP-1 receptor agonist | NA | Acetate ion | NA | NA | NA |
| | | 2.64 | | | |
| Mole ratio of halide ion to GLP-1 receptor agonist | Chloride ion | NA | NA | Chloride ion | NA |
| | 17.61 | | | 7.48 | |
| Mole ratio of amino acid to GLP-1 receptor agonist | NA | NA | NA | NA | NA |
| Syringe | Glass | Glass | COP | COP | Glass |

**Table 3. injectable formulations**

| | Embodiment | | | | |
|---|---|---|---|---|---|
| | 11 | 12 | 13 | 14 | 15 |
| Semaglutide (mg) | 2.4 | 2.4 | 2.4 | 1.2 | 1.2 |
| Semaglutide (wt. %) | 0.38 | 0.38 | 0.45 | 0.51 | 0.49 |
| Soybean phosphatidylcholine (wt. %) | 27.1 | 27.1 | 31.7 | 42.9 | 42.4 |
| Dioleoyl phosphatidyl ethanolamine (wt. %) | NA | NA | NA | NA | NA |
| Glycerol dioleate (wt. %) | 40.8 | 40.8 | 47.8 | 42.9 | 42.4 |
| Propylene glycol (wt. %) | 1.4 | 1.4 | 1.4 | 6.9 | 6.5 |
| Glycerol (wt. %) | NA | NA | NA | NA | 5.0 |
| Other solvent | N-methyl-2-pyrrolido ne | N-methyl-2-pyrrolidone | N-methyl-2-pyrrolidone | Ethanol | Ethanol |
| Concentration of PG based on 100 wt. % by weight percentage of N-methyl-2-pyrrolido ne | 4.7 | 4.7 | 7.5 | NA | NA |
| Concentration of PG based on 100 wt. % by weight percentage of Ethanol | NA | NA | NA | 100 | 100 |
| Mole ratio of alkali metal ion to GLP-1 receptor agonist | Sodium ion | Sodium ion | Sodium ion | Sodium ion | Sodium ion |
| | 4.40 | 4.40 | 4.40 | 4.40 | 4.40 |
| Mole ratio of phosphate ion to GLP-1 receptor agonist | 0.04 | 0.04 | 0.04 | 0.04 | 0.04 |
| Mole ratio of organic acid ion to GLP-1 receptor agonist | Citrate ion | Tartrate ion | NA | NA | NA |
| | 0.90 | 0.34 | | | |
| Mole ratio of halide ion to GLP-1 receptor agonist | NA | NA | NA | NA | NA |
| Mole ratio of amino acid to GLP-1 receptor agonist | NA | NA | NA | NA | NA |
| Syringe | Glass | Glass | Glass | Glass | Glass |

**Table 4. injectable formulations**

| | Comparative Embodiment | | |
|---|---|---|---|
| | 1 | 2 | 3 |
| Semaglutide (mg) | 0.4 | 2.4 | 2.4 |
| Semaglutide (wt. %) | 0.07 | 0.44 | 1.17 |
| Soybean phosphatidylcholine (wt. %) | 31.8 | 31.4 | NA |
| Dioleoyl phosphatidyl ethanolamine (wt. %) | NA | NA | 39.0 |
| Glycerol dioleate (wt. %) | 48.0 | 47.3 | 39.0 |
| Propylene glycol (wt. %) | 1.4 | 1.4 | 9.9 |
| Glycerol (wt. %) | NA | NA | NA |
| Other solvent | N-methyl-2-pyrroli done | N-methyl-2-pyrroli done | Ethanol |
| Concentration of PG based on 100 wt. % by weight percentage of N-methyl-2-pyrrolidone | 7.5 | 7.6 | NA |
| Concentration of PG based on 100 wt. % by weight percentage of Ethanol | NA | NA | 100 |
| Mole ratio of alkali metal ion to GLP-1 receptor agonist | Sodium ion | Sodium ion | Sodium ion |
| | 4.40 | 30.82 | 4.40 |
| Mole ratio of phosphate ion to GLP-1 receptor agonist | 0.04 | 0.04 | 0.04 |
| Mole ratio of organic acid ion to GLP-1 receptor agonist | NA | NA | NA |
| Mole ratio of halide ion to GLP-1 receptor agonist | NA | Chloride ion | NA |
| | | 26.41 | |
| Mole ratio of amino acid to GLP-1 receptor agonist | NA | NA | NA |
| Syringe | Glass | Glass | Glass |

### Measurement method of viscosity of injectable formulation

A viscosity of the injectable formulation was measured by using an Anton-Paar rheometer (MCR 92) at a shear rate range from 0.001 s⁻¹ to 1000 s⁻¹ under 20 °C with the cone-plate geometry (CP25-1), and the viscosity of injectable formulations were recorded in Fig. 1 and Fig. 2. In comparison to all of embodiments, the viscosity of the injectable formulation of comparative embodiment 3 was more than 1000 mPa·s at the shear rate of 1 s⁻¹, higher than all of the embodiment 1 to the embodiment 15. Compared with dioleoyl phosphatidyl ethanolamine used in comparative embodiment 3, soybean phosphatidyl choline used in the embodiment 1 to the embodiment 14 provided better syringeablility.

### Measurement method of water content of injectable formulation

The water content of the injectable formulation was measured by using a Karl-Fischer titrator (Metrohm). Methanol was used to dissolve the injectable formulation. The dissolved injectable formulation was titrated with Karl-Fischer reagent until a titration end point was reached. The result of the water contents of injectable formulations were listed in table 5.

**Table 5. Water content of injectable formulations**

| | | Water content (%) |
|---|---|---|
| | 1 | 1.30 |
| | 2 | 1.23 |
| | 3 | 1.46 |
| | 4 | 0.55 |
| | 5 | 0.52 |
| Embodiment | 6 | 1.12 |
| | 7 | 1.27 |
| | 8 | 1.25 |
| | 9 | 1.23 |
| | 10 | 1.30 |
| | 11 | 0.50 |
| | 12 | 0.61 |
| Comparative Embodiment | 1 | 0.39 |
| | 2 | 1.42 |
| | 3 | 0.98 |

### Method for evaluating appearance of gel formed by injectable formulation

A 200 µL to 650 µL of the injectable formulation was injected into a 4 mL of phosphate buffered saline (10 mM sodium phosphate, 8.25 mg/mL sodium chloride, pH 7.4) at 25 °C through a pipette tip, and then stood for 20 minutes to 30 minutes to form a gel. The gel was visually inspected to evaluate its appearance. As shown in the table 6 below, the appearance of the gel formed by the injectable formulation of the comparative embodiment 2 was defective. Compared with higher mole ratio (more than 20) of chloride ion to the semaglutide used in the comparative embodiment 2, lower mole ratio (not more than 20) of chloride ion to the semaglutide used in all of the embodiment 1 to the embodiment 15 provided an intact appearance of gel. The intact appearance of the gel extended the release period of the GLP-1 receptor agonist from the gel.

**Table 6. Appearance of gel formed by injectable formulations**

| | | Appearance of gel |
|---|---|---|
| | 1 | intact |
| | 2 | intact |
| | 3 | intact |
| | 4 | intact |
| | 5 | intact |
| | 6 | intact |
| Embodiment | 7 | intact |
| | 8 | intact |
| | 9 | intact |
| | 10 | intact |
| | 11 | intact |
| | 12 | intact |
| | 13 | intact |
| | 14 | intact |
| | 15 | intact |
| Comparative Embodiment | 1 | intact |
| | 2 | defective |
| | 3 | intact |

Method for determining residual rate of semaglutide retained within gel in dissolution medium at 37 °C over 20 days or 31 days
The injectable formulation was loaded into the basket in 500 mL of dissolution medium (50 mM potassium phosphate buffer, pH7.4) at 37 °C through a 29 G needle. The loaded formulation was allowed to stand in 37 °C for 20 minutes in order to form a gel. After the gel formation, the basket was span at 100 rpm. After 20 days or 31 days, the gel was taken from the basket and was completely dissolved in benzyl alcohol under ultrasonic treatment, allowing semaglutide within the gel dissolve thoroughly in benzyl alcohol. Then, the dissolved semaglutide was analyzed by High-Performance Liquid Chromatography (HPLC) or Ultra-Performance Liquid Chromatography (UPLC) with gradient elution. A guard column (C18, 1.7 µm, 2.1 × 5 mm) and an analytical column (C18, 1.7 µm, 2.1 × 150 mm) were used. A mobile phase consisted of mobile phase A (trifluoroacetic acid, acetonitrile and water in a volume ratio of 1.5 : 10 : 988.5) and mobile phase B (trifluoroacetic acid and acetonitrile in a volume ratio of 1.5 : 998.5). A detection was performed by using a photo diode array detector at 220 nm. The residual rate (%) of semaglutide within the gel was calculated based on an original weight of semaglutide of the injectable formulation being 100 weight percentages.

According to the following table 7, compared with all of the embodiment 1 to the embodiment 12, a low amount (0.4 mg) of the semaglutide was used in the injectable formulation of comparative embodiment 1, the residual rate of the semaglutide within the gel in the dissolution medium over 31 days was decreased.

Besides, as stated above, according to table 6, the appearance of the gel formed by the injectable formulation of the comparative embodiment 2 was defective. Therefore, in the comparative embodiment 2, the semaglutidewas expected to be easily released from the gel, such that its residual rate was expected to be decreased. Moreover, based on the result of the embodiment 6 and the embodiment 9 (referring to the following table 7), with a decrease of the amount of chloride ion, the residual rate of the semaglutide within the gel was increased.

From data of embodiment 7 and embodiment 8 in the following table 7, compared with the acetate ion, the sodium ion and the phosphate ion increased a residual rate of the semaglutide within the gel, thereby extending a release period of the semaglutide from the gel.

Based on the result of embodiment 8 and embodiment 11, in comparison with the citrate ion, the sodium ion and the phosphate ion increased a residual rate of the semaglutide within the gel, thereby extending a release period of the semaglutide from the gel.

Besides, according to result of embodiment 8 and embodiment 12, compared with the tartrate ion, the sodium ion and the phosphate ion increased a residual rate of the semaglutide within the gel, thereby extending a release period of the semaglutide from the gel. Moreover, all of ingredients used in an embodiment 16 were the same as those used in the embodiment 4, but weights thereof used in the embodiment 16 were 2.8 times as those of the embodiment 4, respectively. In the embodiment 16, a residual rate of semaglutide retained within a gel in a dissolution medium at 37 °C over 30 days also were determined, and the residual rate was 66.40 %.

**Table 7. Residual rate of semaglutide retained within gel in dissolution medium at 37 °C over 20 days or 31 days**

| Residual rate of semaglutide (wt. %) | | 20 days | 31 days |
|---|---|---|---|
| | 1 | NA | 60.38 |
| | 2 | NA | 63.79 |
| | 3 | NA | 64.83 |
| | 4 | 77.30 | 61.59 |
| Embodiment | 5 | NA | 74.10 |
| | 6 | NA | 44.54 |
| | 7 | 53.10 | NA |
| | 8 | 67.41 | NA |
| | 9 | NA | 24.32 |
| | 10 | 31.94 | NA |
| | 11 | 29.47 | NA |
| | 12 | 66.78 | NA |
| Comparative Embodiment | 1 | NA | 23.02 |

| | | | |
|---|---|---|---|
| Note: a term "NA" in table 7 indicated that the condition was not tested. | | | |

### Test method of thermal stability of injectable formulation

The injectable formulation underwent six consecutive cycles of freezing at -20 °C followed by thawing at 25 °C in 60 % RH. The thermal stability of the injectable formulation was performed by gradient HPLC or UPLC. A guard column (C18, 1.7 am, 2.1 × 5 mm) and an analytical column (C18, 1.7 µm, 2.1 × 150 mm) were used. A mobile phase consists of mobile phase A (trifluoroacetic acid, acetonitrile and water in a volume ratio of 1.5 : 10 : 988.5) and mobile phase B (trifluoroacetic acid and acetonitrile in a volume ratio of 1.5 : 998.5). A detection was carried out at 220 nm with a photo diode array detector. An appearance of the injectable formulation was visually inspected prior to cycle 1 and after each subsequent cycle. In all of the embodiments, the appearances of these injectable formulations were clear, pale yellow and exhibited good fluidity after the six consecutive cycles. Besides, there were no gelation and no stratification in these injectable formulations of all of the embodiments. Furthermore, assays of semaglutide of the injectable formulation before and after the consecutive cycles were calculated, and assay differences of semaglutide of injectable formulations were summarized in table 8 below, in which assay difference (%) = (assay (%) of semaglutide after the consecutive cycles) - (assay (%) of semaglutide before the consecutive cycles). The assay differences were used to estimate thermal stability of the injectable formulations.

**Table 8. Thermal stability of injectable formulations after the six consecutive cycles**

| | | Assay Difference (%) |
|---|---|---|
| | 1 | -6.28 |
| | 2 | -4.03 |
| | 3 | -4.80 |
| | 4 | 0.98 |
| | 5 | -3.8 |
| | 6 | -16.90 |
| Embodiment | 7 | -10.50 |
| | 8 | -11.60 |
| | 9 | -13.48 |
| | 10 | -8.32 |
| | 11 | -16.06 |
| | 12 | -16.26 |
| | 13 | -3.01 |
| | 14 | -0.90 |
| Comparative Embodiment | 1 | -17.98 |

According to table 8, compared with the comparative embodiment 1, in the embodiment 1 to the embodiment 14, there were less variations in the assay differences of the injectable formulations. Therefore, their injectable formulations exhibited better thermal stability than that of the comparative embodiment 1.

### Pharmacokinetics studies

The injectable formulations (hereinafter referred to as injects) of embodiment 8, embodiment 13, embodiment 14 and comparative embodiment 3 were injected subcutaneously to sprague dawley rats (each group, n=3) at a dose of 3.75 mg/kg and 7.5 mg/kg, respectively.

Blood of sprague dawley rats for pharmacokinetics were collected pre-dose, and at predetermined sampling points after dosing. Blood samples were placed on ice after collection and then centrifuged, to obtain plasma in each blood sample. The plasma was stored below -70°C. The plasma was analyzed by liquid chromatography-tandem mass spectrometry (LC-MS/MS). Pharmacokinetic profiles for these embodiments and comparative embodiment 3 were recorded.

In comparison with the comparative embodiment 3, in embodiment 8 and embodiment 13, the semaglutide released in vivo from the gel over 28 days. Specifically, following a one-month administration period in a rat model, embodiment 8 and embodiment 13 both achieved a remarkable effect, resulting in a body weight reduction exceeding 20% and marked decrease in food consumption when compared to both the comparative embodiment 3 and the placebo group.

In the comparative embodiment 3, the concentration of semaglutide in blood of the sprague dawley rats was about 0 ng/mL both on 20th and 30th day. In the embodiment 8, the concentration of semaglutide in blood of the sprague dawley rats was 259 ng/mL and 230 ng/mL on 20th and 30th day, respectively. In the embodiment 13, the concentration of semaglutide in blood of the sprague dawley rats was 182 ng/mL and 169 ng/mL on 20th and 30th day, respectively.

In embodiment 14, the semaglutide released in vivo from the gel formed by the injectable formulation over 20 to 28 days. In the embodiment 14, the concentration of semaglutide in blood of the sprague dawley rats was 154 ng/mL and 73 ng/mL on 20th and 30th day, respectively. Surprisingly, the maximum concentration (Cₘₐₓ) of the semaglutide in blood of the sprague dawley rats was not more than 15 times of a minimum concentration (Cₘᵢₙ) of the semaglutide in their blood. Following a one-month administration period in a rat model, embodiment 14 achieved a remarkable effect, resulting in marked body weight reduction when compared to the placebo group.

## Claims

1. An injectable formulation for a single administration, **characterized in that** the injectable formulation comprises:
phosphatidyl choline;
diacyl lipid;
at least one biocompatible organic solvent selected from the group consisting of propylene glycol, N-methyl-2-pyrrolidone, ethanol and glycerol;
a GLP-1 receptor agonist, wherein a concentration of the GLP-1 receptor agonist is in a range of 0.08 wt. % to 5.0 wt. % based on 100 wt. % by weight percentage of the injectable formulation; and
at least one pharmaceutically acceptable ion selected from the group consisting of alkali metal ion and phosphate ion,
wherein the injectable formulation forms a depot upon contacting an aqueous fluid.

2. The injectable formulation for the single administration of claim 1, **characterized in that** the concentration of the GLP-1 receptor agonist is in the range of 0.1 wt. % to 4.0 wt. % based on 100 wt. % by weight percentage of the injectable formulation.

3. The injectable formulation for the single administration of claim 1 or claim 2, **characterized in that** the GLP-1 receptor agonist has a peptide backbone and a side chain bonded thereto, the peptide backbone consists of 28 to 40 amino acids, and the side chain has a structure represented by formula I: 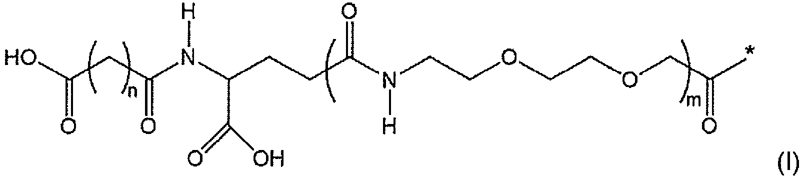 wherein n is an integer from 16 to 24, m is an integer from 0 to 3, * is an attachment point connecting a carbonyl group of the side chain to an amino group of one amino acid of the 28 to 40 amino acids in the peptide backbone.

4. The injectable formulation for the single administration of any one of claims 1 to 3, **characterized in that** a mole ratio of the alkali metal ion to the GLP-1 receptor agonist is more than zero and not more than 25.

5. The injectable formulation for the single administration of any one of claims 1 to 4, **characterized in that** a mole ratio of the phosphate ion to the GLP-1 receptor agonist is 0.01 to 1.2.

6. The injectable formulation for the single administration of any one of claims 1 to 5, **characterized in that** the injectable formulation further comprises organic acid ion with a molecular weight less than 200 g/mole, halide ion, an alkaline amino acid or a combination thereof.

7. The injectable formulation for the single administration of claim 6, **characterized in that** a mole ratio of the alkaline amino acid to the GLP-1 receptor agonist is 0.1 to 2.0.

8. The injectable formulation for the single administration of any one of claims 1 to 7, **characterized in that** the injectable formulation further comprises water, wherein a concentration of the water is in a range of zero wt. % to 1.5 wt. % based on 100 wt. % by weight percentage of the injectable formulation.

9. The injectable formulation for the single administration of any one of claims 1 to 8, **characterized in that** a weight ratio of the phosphatidyl choline to the GLP-1 receptor agonist is from 10 to 85.

10. The injectable formulation for the single administration of any one of claims 1 to 9, **characterized in that** a weight ratio of the diacyl lipid to the GLP-1 receptor agonist is from 3 to 110.

11. An injectable formulation for use in treating a metabolic disease, **characterized in that** the injectable formulation comprises:
a lipid matrix composition, a GLP-1 receptor agonist and at least one pharmaceutically acceptable ion selected from the group consisting of alkali metal ion and phosphate ion, and an amount of the GLP-1 receptor agonist is 1.0 mg to 20 mg, wherein the injectable formulation is administered for a single administration to a subject once every 20 to 45 days.

12. The injectable formulation for use in treating the metabolic disease of claim 11, **characterized in that** the metabolic disease is selected from the group consisting of type 1 diabetes mellitus, type 2 diabetes mellitus, obesity, non-alcoholic steatohepatitis (NASH), non-alcoholic fatty liver disease (NAFLD) and cardiovascular diseases.

13. The injectable formulation for use in treating the metabolic disease of claim 11 or claim 12, **characterized in that** the lipid matrix composition is composed of phosphatidyl choline, diacyl lipid and at least one biocompatible organic solvent.

14. An administration device, **characterized in that** the administration device comprises:
a vial, a pre-filled syringe or a pre-filled cartridge filled with an injectable formulation for a single administration,
wherein the injectable formulation comprises a lipid matrix composition, a GLP-1 receptor agonist and at least one pharmaceutically acceptable ion selected from the group consisting of alkali metal ion and phosphate ion, and a concentration of the GLP-1 receptor agonist is in a range of 0.08 wt. % to 5.0 wt. % based on 100 wt. % by weight percentage of the injectable formulation.

15. The administration device of claim 14, **characterized in that** the vial, the pre-filled syringe and the pre-filled cartridge are made of glass or of plastics material.
